# EUROPÄISCHE PATENTSCHRIFT

(11) **EP 0 584 692 B1**
(45) Veröffentlichungstag und Bekanntmachung des Hinweises auf die Patenterteilung: **16.06.1999**
(21) Anmeldenummer: 93113129.6
(22) Anmeldetag: 17.08.1993
(51) Int. Cl.: A61K 7/50, A61K 7/06, A61K 7/48, C11D 1/62

(54) **Mittel zum Reinigen und Konditionieren der Haare, der Haut sowie von Textilien und harten Oberflächen**
Cleaning and conditioning agent for hair, skin as well as for textiles and hard surfaces
Produit de nettoyage et de conditionnement pour les cheveux, la peau, ainsi que les textiles et les surfaces dures

(30) Priorität: 27.08.1992 DE 4228594
(43) Veröffentlichungstag der Anmeldung: 02.03.1994
(73) Patentinhaber: MÄURER + WIRTZ GmbH & Co. KG, D-52224 Stolberg (DE)
(72) Erfinder: Müller, Wilfried, D-52531 Übach-Palenberg (DE); Vathje, Rainer, Dipl.-Ing., D-52223 Stolberg (DE)
(74) Vertreter: Patentanwälte Sternagel & Fleischer

(56) Entgegenhaltungen:
- EP-A- 0 282 864
- DE-A- 2 129 724
- DE-C- 4 314 678
- FR-A- 2 125 530

## Beschreibung

Die Erfindung betrifft wäßrige und/oder alkoholische Mittel mit einem Gehalt an Tensiden zur Reinigung und Konditionierung der Haare, der Haut sowie von Textilien und harten Oberflächen und ein Verfahren zur Herstellung dieser Mittel.

Stark schäumende Körperwasch- und Reinigungsmittel enthalten als Hauptbestandteil Tenside, die die Haut stark entfetten und ein unangenehmes Gefühl nach der Anwendung hinterlassen. Um diesem Effekt entgegen zu wirken, werden beispielsweise Fettsäurealkylolamide als Rückfettungsmittel (Konditioniermittel) eingesetzt. Aufgrund toxikologischer Bedenken wurde jedoch der Einsatz dieser Substanzen stark limitiert.

Neben dem Einsatz der vorgenannten Konditioniermittel, die in dem umgebenden Medium löslich sind und keinerlei Probleme bei der Lagerung bei Umgebungstemperatur bewirken, ist auch die Verwendung von wasserunlöslichen, hydrophoben Komponenten, beispielsweise Fettsäureestern, langkettigen Fettalkoholen und/oder hochmolekularen Kohlenwasserstoffen als Konditioniermittel in tensidhaltigen Mitteln bekannt. Die hydrophoben Komponenten haben jedoch einen negativen Einfluß sowohl auf die Schaumkraft als auch auf die Lagerstabilität tensidhaltiger Mittel. Der unerwünschte Einfluß hinsichtlich der Lagerstabilität macht sich insbesondere durch Phasentrennungen und irreversible Viskositätsveränderungen bemerkbar.

Um zu erreichen, daß bei tensidhaltigen Mitteln, die wasserunlösliche und/oder in Wasser schwerlösliche Bestandteile enthalten, sowohl bei niedrigen Temperaturen (<10° C) als auch bei höheren Temperaturen (>35° C) weder Phasentrennungen noch irreversible Viskositätsveränderungen auftreten, ist ein sehr hoher Tensidgehalt (>25 Gew.-%) und/oder die Zugabe von Verdickungsmitteln erforderlich. Diese Mittel haben jedoch bei Temperaturen zwischen 18 und 25° C in Bezug auf Schaumverhalten und Hautgefühl unangenehme Gebrauchseigenschaften. Darüber hinaus kommt es bei Temperaturen oberhalb von etwa 35° C sowie bei Temperaturen unterhalb von etwa 10° C häufig zu Phasentrennungen.

Es wurde nunmehr gefunden, daß Kombinationen aus
a) C₃₆-Dimerfettsäure und/oder C₅₄-Trimerfettsäure,
b) einem C₁₀₋₁₆-Alkanol und/oder einem C₁₀₋₁₆-Alkandiol mit endständigen, vicinalen Hydroxylgruppen und
c) einer bestimmten quartäre Ammoniumgruppen enthaltenden Verbindung
in bestimmten Mengen zugesetzt, Mitteln mit einem Tensidgehalt zwischen 10 und 25 Gew.-% bei Temperaturen zwischen etwa 4° C und 60° C eine hervorragende Lagerstabilität verleihen. Im Vergleich zu bekannten tensidhaltigen Mitteln besitzen die erfindungsgemäßen Mittel bessere Schaumeigenschaften und führen zu einer signifikanten Verbesserung des Hautgefühls. Aufgrund ihrer flüssigen Konsistenz in einem Viskositätsbereich zwischen 700 mpas und 6.000 mpas, gemessen mit einem Rotationsviskosimeter der Fa. Haake, Typ RV20-M10 (Spindel SV DIN; Umdrehungsgeschwindigkeit: 30/Minute) bei Temperaturen zwischen etwa 4° C und 60° C, eignen sich die erfindungsgemäßen Mittel hervorragend für die Reinigung und Konditionierung der Haare, der Haut sowie von Textilien und harten Oberflächen.

Erfindungsgegenstand sind dementsprechend wäßrige und/oder alkoholische Mittel mit einem Gehalt an Tensiden für die Reinigung und Konditionierung der Haare, der Haut sowie von Textilien und harten Oberflächen, welche dadurch gekennzeichnet sind, daß
a) 0,1 bis 10 Gew.-% C₃₆-Dimerfettsäure und/oder C₅₄-Trimerfettsäure,
b) 0,1 bis 5 Gew.-% wenigstens eines C₁₀₋₁₆-Alkanols und/oder wenigstens eines C₁₀₋₁₆-Alkandiols mit endständigen, vicinalen Hydroxylgruppen und
c) 0,1 bis 5 Gew.-% wenigstens einer quartären Ammoniumverbindung der Formel 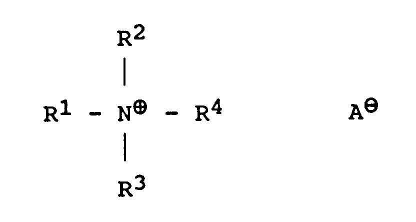 in der 1 bis 3 der Reste R¹ bis R⁴ Methyl- und/oder Hydroxyethylgruppen und 1 bis 3 der Reste R¹ bis R⁴ C₁₀₋₂₂-Fettalkyl- und/oder C₁₀₋₂₂-Fettalkenylgruppen darstellen und A^{⊖} ein Chlorid-, Bromid-, Methoxysulfat-, Hydrogensulfat-, Sulfat-, Hydrogenphosphat-, Phosphat-, Lactat- oder Citratanion bedeutet, und/oder wenigstens eines quartäre Ammoniumgruppen enthaltenden Cellulose-, Guar-, Zucker- und/oder Xanthanderivates und/oder wenigstens eines quarternisierten Proteins
   enthalten sind.

Vorzugsweise enthält ein erfindungsgemäßes Mittel 0,3 bis 5 Gew.-% an Komponente a), 1 bis 3 Gew.-% an Komponente b) und 0,3 bis 2 Gew.-% an Komponente c). Die durch thermische En-Addition aus ungesättigten C₁₈-Fettsäuren, beispielsweise aus Ölsäure und/oder Linolsäure zugängliche C₃₆-Dimerfettsäure bzw. C₅₄-Trimerfettsäure kann als reine Dimerfettsäure bzw. reine Trimerfettsäure sowie als Mischung aus Dimer- und Trimerfettsäure vorliegen. Vorzugsweise werden C₃₆-Dimerfettsäure und/oder C₅₄-Trimerfettsäure mit einer Jodzahl zwischen 5 und 15 eingesetzt, beispielsweise die im Handel unter der Bezeichnung Pripol® 1009 Dimersäure, gehärtet erhältliche C₃₆-Dimerfettsäure von Unichema International.

Beispiele geeigneter C₁₀₋₁₆-Alkanole natürlichen und/oder synthetischen Ursprungs und/oder geeigneter C₁₀₋₁₆-Alkandiole mit endständigen, vicinalen Hydroxylgruppen sind Decanol, Dodecanol, Tetradecanol, Hexadecanol, 1,2-Decandiol, 1,2-Dodecandiol, 1,2-Tetradecandiol und/oder 1,2-Hexadecandiol. Bevorzugt werden Decanol, Dodecanl und/oder Tetradecanol eingesetzt.

Als quartäre Ammoniumverbindungen der Formel 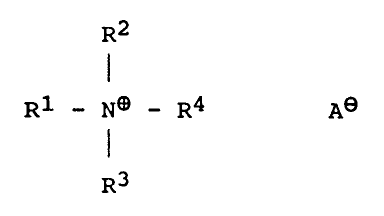 eignen sich bei beispielsweise Cetyltrimethylammoniumchlorid, Distearyldimethylammoniumchlorid und/oder Stearyloxyethylammoniumphosphat. Als quarternisiertes Protein kommt beispielsweise ein alkylquarternisiertes, hydrolysiertes Sojaprotein (Croquat® Soya der Croda GmbH) in Frage. Beispiele der bevorzugt als Komponente c) eingesetzten quartäre Ammoniumgruppen enthaltenen Cellulose-, Guar-, Zucker- und/oder Xanthanderivate sind die im Handel unter der Bezeichnung Crodacel® QM, Crodacel® QL und Crodacel® QS (Fa. Croda GmbH) erhältlichen Hydroxyethylcellulosen Hydroxyethylcellulose-kokosalkyldimethylammoniumchlorid, Hydroxyethylcellulose-lauryldimethylammoniumchlorid und Hydroxyethylcellulose-stearyldimethylammoniumchlorid, das von Meyhall GmbH unter der Bezeichnung Jaguar® C-14 erhältliche Hydroxypropylguar-trimethylammoniumchlorid, das von Union Carbide Corp. unter der Bezeichnung Polymer® JR 125 erhältliche Hydroxyethylcellulose-glyceridyltrimethylammoniumchlorid, das von Union Carbide Corp. unter der Bezeichnung Polymer® LM-200 erhältliche Hydroxyethylcelluloselauryldimethylammoniumchlorid sowie die von Amerchol unter der Bezeichnung Gluquat® 100 erhältliche quartäre Ammoniumgruppe enthaltende ethoxylierte Glucose. Quartäre Ammoniumgruppen enthaltende Hydroxyethylcellulosen werden besonders bevorzugt eingesetzt.

Die erfindungsgemäßen Mittel enthalten vorzugsweise 10 bis 20 Gew.-% anionische, nichtionische und/oder zwitterrionische Tenside. Als anionische Tenside kommen insbesondere Alkali-, Ammonium- und/oder Alkanolammoniumsalze mit 2 bis 4 C-Atomen in der Alkanolgruppe von Alkylethersulfaten mit 10 bis 16 C-Atomen in der Alkylgruppe und 1 bis 12 Glykolethergruppen, C₁₀₋₁₈-Alkansulfonaten und/oder Sulfobernsteinsäuremono- und/oder diestern von C₁₀₋₁₆-Alkylpolyglykolethern, als nichtionische Tenside insbesondere Anlagerungsprodukte von 20 bis 60 Mol Ethylenoxid an C₁₂₋₁₈-Fettalkohole, an C₁₂₋₁₈-Fettsäuren und/oder an C₁₂₋₁₈-Fettsäuremono- und -diglyceride und als zwitterionische Tenside insbesondere N-Hydroxyethyl-N-kokosalkylamidoethylcarboxymethylglycinat als Alkalisalze in Frage.

Als fakultative Bestandteile enthalten die erfindungsgemäßen Mittel Stell- und Hilfsmittel, beispielsweise pH-Wert-Regulatoren, Viskositätsregulatoren und/oder Parfümöle in einer Gesamtmenge zwischen 0,1 und 8 Gew.-%.

Weiterer Erfindungsgegenstand ist ein Verfahren zur Herstellung wäßriger und/oder alkoholischer, Tenside enthaltender Mittel, welches dadurch gekennzeichnet ist, daß man eine Mischung, enthaltend Komponente a), Komponente b) und Komponente c) unter Rühren auf eine Temperatur oberhalb des Schmelzpunktes des am höchsten schmelzenden Bestandteils erwärmt und anschließend unter Rühren in eine wäßrige und/oder alkoholische, Tenside enthaltende Mischung gibt.

Vor Zugabe der essentiellen Bestandteile enthält die tensidhaltige Mischung neben den fakultativen Bestandteilen vorzugsweise nur einen Teil der in den erfindungsgemäßen Mitteln enthaltenden Wassermenge. Erst wenn nach Zugabe der die essentiellen Komponenten a), b) und c) enthaltenden Mischung die tensidhaltige Mischung homogen ist, wird die restliche Wassermenge zugegeben. Der pH-Wert der erfindungsgemäßen Mittel wird mit einer Base, beispielsweise Kalilauge bzw. mit einer Säure, beispielsweise Zitronensäure auf einen Wert zwischen 4,0 und 7,0 eingestellt.

Die im Temperaturbereich zwischen etwa 4° C und 60° C lagerstabilen (keine Phasentrennung nach mindestens einem Monat, die Viskosität zeigt ein lineares Verhalten) erfindungsgemäßen Mittel bilden einen feinporigen, sahnigen Schaum, bewirken eine signifikante Verbesserung des Hautgefühls, erhöhen die Wasserabweisung und verbessern die Weichheit von Textilien. Die erfindungsgemäßen Mittel können dementsprechend beispielsweise als Haarshampoos mit Konditioniereigenschaften, als Duschgele mit besonderer Pflegewirkung, beispielsweise mit Bodylotion, als Schaumbäder mit pflegenden Eigenschaften, als Handwaschlotionen mit Rückfettungseffekten sowie als Wollwaschmittel und als Reinigungsmittel für harte Oberflächen aus beispielsweise Keramik, Glas, Stahl oder PVC eingesetzt werden.

### Beispiele

Die Zahlenangaben der Zusammensetzungen sind Gew.-%-Angaben.

### Duschgel mit pflegenden Komponenten - Verwendung unterschiedlicher C₁₀₋₁₆-Alkanole

Eine Mischung, enthaltend ein C₁₀₋₁₆-Alkanol, C₃₆-Dimerfettsäure und Hydroxyethylcellulose-kokosalkyldimethylammoniumchlorid (Crodacel® QM, der Croda GmbH), wurde unter Rühren auf eine Temperatur oberhalb des Schmelzpunktes des am höchsten schmelzenden Bestandteils erwärmt und in eine wäßrige, tensidhaltige Mischung, in der 3/4 der Wassermenge des herzustellenden Mittels enthalten waren, eingerührt. Nachdem eine homogene Mischung vorlag, wurde die restliche Wassermenge zugegeben und homogen gerührt. Der pH-Wert wurde mit verdünnter Kalilauge bzw. Zitronensäure auf einen Wert zwischen 5,2 und 5,5 eingestellt.

In Tabelle 1 sind die Zusammensetzungen der hergestellten erfindungsgemäßen Mittel zusammengefaßt. Bei den erfindungsgemäßen Mitteln wurden nach einem Monat bei 50 °C keine Phasentrennungen beobachtet, wohingegen sich bei den zum Vergleich hergestellten Mittel spätestens nach zwei Wochen bei 50° C hydrophobe Bestandteile als separate obere Phase abschieden.

**Tabelle 1**

| Zusammensetzung | Beispiel | | | | Vergleich | |
|---|---|---|---|---|---|---|
| | 1 | 2 | 3 | 4 | V1 | V2 |
| Decanol | 2,00 | | | | | |
| Dodecanol | | 2,00 | | | | |
| Tetradecanol | | | 2,00 | | | |
| Hexadecanol | | | | 2,00 | | |
| Octadecanol | | | | | 2,00 | |
| Docosanol | | | | | | 2,00 |
| Oleylalkohol | 0,10 | 0,10 | 0,10 | 0,10 | 0,10 | 0,10 |
| Pripol® 1009 Dimersäure gehärtet | 3,00 | 3,00 | 3,00 | 3,00 | 3,00 | 3,00 |
| Crodacel® QM | 2,00 | 2,00 | 2,00 | 2,00 | 2,00 | 2,00 |
| 1,3-Butandiol | 2,50 | 2,50 | 2,50 | 2,50 | 2,50 | 2,50 |

| Tensidhaltige Mischung | | | | | | |
|---|---|---|---|---|---|---|
| Entsalztes Wasser | 55,44 | 55,44 | 55,44 | 55,44 | 55,44 | 55,44 |
| Texapon® N70 (1) | 16,51 | 16,51 | 16,51 | 16,51 | 16,51 | 15,51 |
| Texapon® SB3 (2) | 7,07 | 7,07 | 7,07 | 7,07 | 7,07 | 7,07 |
| Magnesiumchlorid x 6 Kristallwasser | 1,31 | 1,31 | 1,31 | 1,31 | 1,31 | 1,31 |
| Kaliumsorbat | 0,20 | 0,20 | 0,20 | 0,20 | 0,20 | 0,20 |
| Natriumformiat | 0,22 | 0,22 | 0,22 | 0,22 | 0,22 | 0,22 |
| Rewoteric® AM 2C NM (3) | 8,10 | 8,10 | 8,10 | 8,10 | 8,10 | 8,10 |
| Zitronensäure krist. | 0,50 | 0,50 | 0,50 | 0,50 | 0,50 | 0,50 |
| Kalilauge ca. 50 % | 0,25 | 0,25 | 0,25 | 0,25 | 0,25 | 0,25 |
| Parfümöl | 0,80 | 0,80 | 0,80 | 0,80 | 0,80 | 0,80 |
| pH-Wert bei 20° C | 5,20 | 5,30 | 5,20 | 5,10 | 5,20 | 5,30 |
| Viscosität bei 20° C in mpas | 2375 | 3750 | 2210 | 4080 | 1000 | 1300 |

| | | | | | | |
|---|---|---|---|---|---|---|
| (1) = Natriumlaurylethersulfat der Henkel KGaA | | | | | | |
| (2) = Dinatriumlaurylpolyglycolethersulfosuccinat der Henkel KGaA | | | | | | |
| (3) = N-Hydroxyethyl-N-kokosalkylamidoethyl-carboxymethylglycinat, Na-Salz der Rewo Chemische Werke GmbH | | | | | | |

### Duschgel mit pflegenden Komponenten - Verwendung unterschiedlicher C₁₀₋₁₆-Alkandiole

Die erfindungsgemäßen Mittel wurden wie in Beispiel 1 beschrieben hergestellt. Anstelle eines C₁₀₋₁₆-Alkanols wurde ein C₁₀₋₁₆-Alkandiol mit endständigen, vicinalen Hydroxylgruppen eingesetzt.

Die Zusammensetzungen der hergestellten erfindungsgemäßen Mittel sind in Tabelle 2 zusammengefaßt. Bei den erfindungsgemäßen Mitteln wurden nach einem Monat bei 50 °C keine Phasentrennungen beobachtet, wohingegen sich bei den zum Vergleich hergestellten Mittel spätestens nach zwei Wochen bei 50° C hydrophobe Bestandteile als separate obere Phase abschieden.

**Tabelle 2**

| Zusammensetzung | Beispiel | | | | Vergleich | | |
|---|---|---|---|---|---|---|---|
| | 5 | 6 | 7 | 8 | V3 | V4 | V5 |
| 1,2-Octandiol | | | | | 2,00 | | |
| 1,2-Decandiol | 2,00 | | | | | | |
| 1,10-Decandiol | | | | | | 2,00 | |
| 1,2-Dodecandiol | | 2,00 | | | | | |
| 1,12-Dodecandiol | | | | | | | 2,00 |
| 1,2-Tetradecandiol | | | 2,00 | | | | |
| 1,2-Hexadecandiol | | | | 2,00 | | | |
| Oleylalkohol | 0,10 | 0,10 | 0,10 | 0,10 | 0,10 | 0,10 | 0,10 |
| Pripol® 1009 Dimersäure gehärtet | 3,00 | 3,00 | 3,00 | 3,00 | 3,00 | 3,00 | 3,00 |
| Crodacel® QM | 2,00 | 2,00 | 2,00 | 2,00 | 2,00 | 2,00 | 2,00 |
| 1,3-Butandiol | 2,50 | 2,50 | 2,50 | 2,50 | 2,50 | 2,50 | 2,50 |

| Tensidhaltige Mischung | | | | | | | |
|---|---|---|---|---|---|---|---|
| Entsalztes Wasser | 55,44 | 55,44 | 55,44 | 55,44 | 55,44 | 55,44 | 55,44 |
| Texapon® N70 | 16,51 | 16,51 | 16,51 | 16,51 | 16,51 | 16,51 | 15,51 |
| Texapon® SB3 | 7,07 | 7,07 | 7,07 | 7,07 | 7,07 | 7,07 | 7,07 |
| Magnesiumchlorid x 6 Kristallwasser | 1,31 | 1,31 | 1,31 | 1,31 | 1,31 | 1,31 | 1,31 |
| Kaliumsorbat | 0,20 | 0,20 | 0,20 | 0,20 | 0,20 | 0,20 | 0,20 |
| Natriumformiat | 0,22 | 0,22 | 0,22 | 0,22 | 0,22 | 0,22 | 0,22 |
| Rewoteric® AM 2C NM | 8,10 | 8,10 | 8,10 | 8,10 | 8,10 | 8,10 | 8,10 |
| Zitronensäure krist. | 0,50 | 0,50 | 0,50 | 0,50 | 0,50 | 0,50 | 0,50 |
| Kalilauge ca. 50 % | 0,25 | 0,25 | 0,25 | 0,25 | 0,25 | 0,25 | 0,25 |
| Parfümöl | 0,80 | 0,80 | 0,80 | 0,80 | 0,80 | 0,80 | 0,80 |
| pH-wert bei 20° C | 5,30 | 5,20 | 5,40 | 5,10 | 5,10 | 5,20 | 5,30 |
| Viscositat bei 20° C in mpas | 1220 | 1500 | 1030 | 1235 | 1740 | 1660 | 1520 |

### Duschgel mit pflegenden Komponenten - Verwendung unterschiedlicher, Ammoniumgruppen enthaltender Hydroxyethylcellulosen

Die erfindungsgemäßen Mittel wurden wie in Beispiel 1 beschrieben hergestellt. Die Zusammensetzungen der erhaltenen Mittel sind in Tabelle 3 zusammengefaßt. Bei keinem der erfindungsgemäßen Mittel wurde nach einer 1-monatigen Lagerung bei 50° C eine Phasentrennung beobachtet.

**Tabelle 3**

| Zusammensetzung | Beispiel | | | | | | |
|---|---|---|---|---|---|---|---|
| | 9 | 10 | 11 | 12 | 13 | 14 | 15 |
| Crodacel® QL | 2,27 | | | | | | |
| Crodacel® QS | | 2,27 | | | | | |
| Jaguar® C-14 | | | 0,50 | | | | |
| Polymer JR 125 | | | | 0,50 | | | |
| Polymer LM-200 | | | | | 0,50 | | |
| Gluquat® 100 | | | | | | 0,50 | |
| Croquat® Soya | | | | | | | 1,67 |
| Tetradecanol | 2,00 | 2,00 | 2,00 | 2,00 | 2,00 | 2,00 | 2,00 |
| Oleylalkohol | 0,10 | 0,10 | 0,10 | 0,10 | 0,10 | 0,10 | 0,10 |
| Pripol® 1009 Dimersäure gehärtet | 3,00 | 3,00 | 3,00 | 3,00 | 3,00 | 3,00 | 3,00 |
| 1,3-Butandiol | 2,50 | 2,50 | 2,50 | 2,50 | 2,50 | 2,50 | 2,50 |

| Tensidhaltige Mischung | | | | | | | |
|---|---|---|---|---|---|---|---|
| Entsalztes Wasser | 55,17 | 55,17 | 56.94 | 56,94 | 56,94 | 56,94 | 55,77 |
| Texapon® N70 | 16,51 | 16,51 | 16,51 | 16,51 | 16,51 | 16,51 | 15,51 |
| Texapon® SB3 | 7,07 | 7,07 | 7,07 | 7,07 | 7,07 | 7,07 | 7,07 |
| Magnesiumchlorid x 6 Kristallwasser | 1,31 | 1,31 | 1,31 | 1,31 | 1,31 | 1,31 | 1,31 |
| Kaliumsorbat | 0,20 | 0,20 | 0,20 | 0,20 | 0,20 | 0,20 | 0,20 |
| Natriumformiat | 0,22 | 0,22 | 0,22 | 0,22 | 0,22 | 0,22 | 0,22 |
| Rewoteric® AM 2C NM | 8,10 | 8,10 | 8,10 | 8,10 | 8,10 | 8,10 | 8,10 |
| Zitronensäure krist. | 0,50 | 0,50 | 0,50 | 0,50 | 0,50 | 0,50 | 0,50 |
| Kalilauge ca. 50 % | 0,25 | 0,25 | 0,25 | 0,25 | 0,25 | 0,25 | 0,25 |
| Parfümöl | 0,80 | 0,80 | 0,80 | 0,80 | 0,80 | 0,80 | 0,80 |
| pH-Wert bei 20° C | 5,40 | 5,30 | 5,10 | 5,40 | 5,40 | 5,50 | 5,30 |
| Viscositat bei 20° C in mpas | 4100 | 3200 | 2350 | 850 | 3050 | 700 | 820 |

### Duschgel mit pflegenden Komponenten - Herstellung tensidhaltiger Mittel, in denen eine essentielle Komponente fehlt

Die tensidhaltigen Mittel wurden wie in Beispiel 1 beschrieben hergestellt. Die Zusammensetzungen der Mittel sind in Tabelle 4 zusammengestellt. Mit steigender Temperatur nahm die Viskosität drastisch ab (von etwa 4000 mPas bei 20° C auf etwa 700 bis etwa 1500 mPas bei 45° C). Nach einer Lagerung von spätestens 2 Wochen bildeten wasserunlösliche Bestandteile eine separate, obere Phase.

**Tabelle 4**

| Zusammensetzung | Vergleich | | |
|---|---|---|---|
| | V6 | V7 | V8 |
| Tetradecanol | | 2,00 | 2,00 |
| Pripol® 1009 Dimersäure gehärtet | 3,00 | | 3,00 |
| Crodacel® QM | 2,00 | 2,00 | |
| 1,3-Butandiol | 2,50 | 2,50 | 2,50 |
| Oleylalkohol | 0,10 | 0,10 | 0,10 |

| Tensidhaltige Mischung | | | |
|---|---|---|---|
| Entsalztes Wasser | 57,54 | 58,54 | 57,54 |
| Texapon® N70 | 16,51 | 16,51 | 16,51 |
| Texapon® SB3 | 7,07 | 7,07 | 7,07 |
| Magnesiumchlorid x 6 Kristallwasser | 1,31 | 1,31 | 1,31 |
| Kaliumsorbat | 0,20 | 0,20 | 0,20 |
| Natriumformiat | 0,22 | 0,22 | 0,22 |
| Rewoteric® AM 2C NM | 8,10 | 8,10 | 8,10 |
| Zitronensäure krist. | 0,50 | 0,50 | 0,50 |
| Kalilauge ca. 50 % | 0,25 | 0,25 | 0,25 |
| Parfümöl | 0,80 | 0,80 | 0,80 |
| pH-Wert bei 20° C | 5,30 | 5,30 | 5,40 |
| Viscosität bei 20° C in mpas | 4150 | | 3950 |

## Patentansprüche

1. Wäßrige und/oder alkoholische Mittel mit einem Gehalt an Tensiden zur Reinigung und Konditionierung der Haare, der Haut sowie von Textilien und harten Oberflächen, dadurch gekennzeichnet, daß
a) 0,1 bis 10 Gew.-% C₃₆-Dimerfettsäure und/oder C₅₄-Trimerfettsäure,
b) 0,1 bis 5 Gew.-% wenigstens eines C₁₀₋₁₆-Alkanols und/oder wenigstens eines C₁₀₋₁₆-Alkandiols mit endständigen, vicinalen Hydroxylgruppen und
c) 0,1 bis 5 Gew.-% wenigstens einer quartären Ammoniumverbindung der Formel 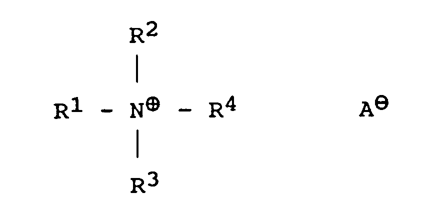 in der 1 bis 3 der Reste R¹ bis R⁴ Methyl- und/oder Hydroxyethylgruppen und 1 bis 3 der Reste R¹ bis R⁴ C₁₀₋₂₂-Fettalkyl- und/oder C₁₀₋₂₂-Fettalkenylgruppen darstellen und A^{⊖} ein Chlorid-, Bromid-, Methoxysulfat-, Hydrogensulfat-, Sulfat-, Phosphat-, Hydrogenphosphat-, Lactat- oder Citratannion bedeutet, und/oder wenigstens eines quartäre Ammoniumgruppen enthaltenden Cellulose-, Guar-, Zucker- und/oder Xanthanderivates und/oder wenigstens eines quarternisierten Proteins
enthalten sind.

2. Mittel nach Anspruch 1, dadurch gekennzeichnet, daß 0,3 bis 5 Gew.-% an Komponente a), 1 bis 3 Gew.-% an Komponente b) und 0,3 bis 2 Gew.-% an Komponente c) enthalten sind.

3. Mittel nach einem oder beiden der Ansprüche 1 bis 2, dadurch gekennzeichnet, daß Decanol, Dodecanol und/oder Tetradecanol als Komponente b) enthalten sind.

4. Mittel nach einem oder beiden der Ansprüche 1 bis 2, dadurch gekennzeichnet, daß wenigstens ein quartäre Ammoniumgruppen enthaltendes Cellulose-, Guar-, Zucker- und/oder Xanthanderivat, vorzugsweise eine quartäre Ammoniumgruppen enthaltende Hydroxyethylcellulose als Komponente c) enthalten ist.

5. Verfahren zur Herstellung eines wäßrigen und/oder alkoholischen Mittels mit einem Gehalt an Tensiden nach Anspruch 1, dadurch gekennzeichnet, daß man eine Mischung, enthaltend Komponente a), Komponente b) und Komponente c), unter Rühren auf eine Temperatur oberhalb des Schmelzpunktes des am höchsten schmelzenden Bestandteils erwärmt und anschließend unter Rühren in eine wäßrige und/oder alkoholische, Tenside enthaltende Mischung gibt.

## Claims

1. Aqueous and/or alcohol-based compositions containing surfactants for cleaning and conditioning of hair, skin as well as textiles and rigid surfaces, characterized by comprising:
a) 0.1 to 10 percent by weight C₃₆-dimeric fatty acid and/or C₅₄-trimeric fatty acid,
b) 0.1 to 5 percent by weight of at least one C₁₀₋₁₆-alkanol and/or at least one C₁₀₋₁₆-alkane diol having terminal vicinal hydroxyl groups, and
c) 0.1 to 5 percent by weight of at least one quaternary ammonium compound of the formula 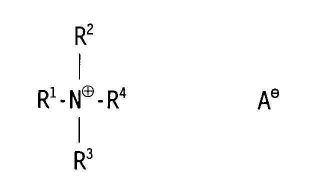 wherein 1 to 3 of the groups R¹ to R⁴ are methyl groups and/or hydroxyethyl groups, and 1 to 3 of the groups R¹ to R⁴ are C₁₀₋₂₂-fatty alkyl groups and/or C₁₀₋₂₂-fatty alkenyl groups, and A^{⊖} is a chloride, bromide, methoxy sulfate, hydrogen sulfate, sulfate, phosphate, hydrogen phosphate, lactate or citrate anion, and/or at least one of quaternary ammonium groups containing cellulose, guar gum, sugar and/or xanthene derivate, and/or at least one quaternized protein.

2. Composition according to claim 1, characterized by comprising 0.3 to 5 percent by weight of component (a), 1 to 3 percent by weight of component (b) and 0.3 to 2 percent by weight of component (c).

3. Composition according to one or both of claims 1 and 2, characterized in that decanol, dodecanol and/or tetradecanol are present as component (b).

4. Composition according to one or both of claims 1 and 2, characterized in that at least one quaternary ammonium group containing cellulose derivative, guaran derivative, sugar derivative and/or xanthen derivative, preferably a quaternary ammonium group containing hydroxy cellulose is present as component (c).

5. Process for the preparation of an aqueous and/or alcohol-based composition comprising a content of surfactants according to claim 1, characterized in that a blend containing component (a), component (b) and component (c) is heated under agitation to a temperature above the melting point of the component having the highest melting point, and is subsequently added under agitation to an aqueous and/or alcohol-based surfactant containing mixture.

## Revendications

1. Produit aqueux et/ou alcoolisé contenant des tensioactifs, qui est destiné au nettoyage et au conditionnement des cheveux, de la peau, ainsi que de textiles et de surfaces dures, caractérisé en ce qu'il contient
a) 0,1 à 10 % en poids d'acide gras (C₃₆)-dimère et/ou d'acide gras (C₅₄)-trimère,
b) 0,1 à 5 % en poids d'au moins un (C₁₀₋₁₆)-alcanol et/ou d'au moins un (C₁₀₋₁₆)-alcanediol ayant des groupes hydroxyle vicinaux en fin de chaîne, et
c) 0,1 à 5 % en poids d'au moins un composé ammonium quaternaire de formule 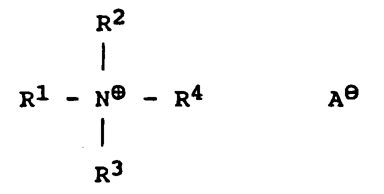 dans laquelle 1 à 3 des radicaux R¹ à R⁴ représentent des groupes méthyle et/ou hydroxyéthyle, et 1 à 3 des radicaux R¹ à R⁴ des groupes alkyle gras en C₁₀₋₂₂ et/ou alkylène gras en C₁₀₋₂₂, et A^{⊖} représente un anion chlorure, bromure, méthoxysulfate, sulfate d'hydrogène, sulfate, phosphate, phosphate d'hydrogène, lactate ou citrate, et/ou au moins un dérivé de cellulose, de guar, de sucre et/ou de xanthane contenant des groupes ammonium quaternaire et/ou au moins une protéine quaternisée.

2. Produit selon la revendication 1, caractérisé en ce qu'il contient 0,3 à 5 % en poids du composé a), 1 à 3 % en poids du composé b), et 0,3 à 2 % en poids du composé c).

3. Produit selon l'une ou les deux revendications 1 et 2, caractérisé en ce qu'il contient en tant que composé b) du décanol, du dodécanol, et/ou du tétradécanol.

4. Produit selon l'une ou les deux revendications 1 et 2, caractérisé en ce qu'il contient en tant que composé c) au moins un dérivé de cellulose, de guar, de sucre et/ou de xanthane contenant des groupes ammonium quaternaire, de préférence une hydroxyéthylcellulose contenant des groupes ammonium quaternaire.

5. Procédé destiné à la fabrication d'un produit aqueux et/ou alcoolisé contenant des tensioactifs selon la revendication 1, caractérisé en ce qu'un mélange contenant le composé a), le composé b), et le composé c) est porté sous agitation à une température supérieure au point de fusion de l'ingrédient ayant le point de fusion le plus élevé, et qu'il est ensuite ajouté sous agitation à un mélange aqueux et/ou alcoolisé contenant des tensioactifs.
